(19) Europäisches Patentamt
European Patent Office
Office européen des brevets

(11) Veröffentlichungsnummer: **0 029 908**
**B1**

# (12) EUROPÄISCHE PATENTSCHRIFT

(45) Veröffentlichungstag der Patentschrift: **22.12.82**

(51) Int. Cl.³: **A 01 N 43/72,** C 07 D 285/00 // C07C143/833, C07C127/26

(21) Anmeldenummer: **80106394.2**

(22) Anmeldetag: **21.10.80**

(54) **6H-1,2,4,6-Thiatriazin-1,1-dioxide, Herbizide, die diese Verbindungen enthalten, ihre Anwendung als Herbizide und Verfahren zu ihrer Herstellung.**

(30) Priorität: **30.10.79 DE 2943703**

(43) Veröffentlichungstag der Anmeldung: **10.06.81 Patentblatt 81/23**

(45) Bekanntmachung des Hinweises auf die Patenterteilung: **22.12.82 Patentblatt 82/51**

(84) Benannte Vertragsstaaten: **AT BE CH DE FR GB IT LI NL SE**

(56) Entgegenhaltungen:
**DE-A-1 946 262**
**DE-A-2 026 625**
**DE-A-2 508 832**

(73) Patentinhaber: **BASF Aktiengesellschaft, Carl-Bosch-Strasse 38, D-6700 Ludwigshafen (DE)**

(72) Erfinder: **Hamprecht, Gerhard, Dr., Rote-Turm-Strasse 28, D-6940 Weinheim (DE)**
Erfinder: **Acker, Rolf-Dieter, Dr., Tuchbleiche 8, D-6906 Leimen (DE)**
Erfinder: **Wuerzer, Bruno, Dr., Ruedigerstrasse 13, D-6701 Otterstadt (DE)**

**EP 0 029 908 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 6H-1,2,4,6-Thiatriazin-1,1-dioxide, Herbizide, die diese Verbindungen enthalten, ihre Anwendung als Herbizide und Verfahren zu ihrer Herstellung

Die vorliegende Erfindung betrifft neue 6H-1,2,4,6-Thiatriazin-1,1-dioxide, Verfahren zur Herstellung dieser Verbindungen, sowie Herbizide, welche diese Verbindungen enthalten und ihre Anwendung als Herbizide.

Es ist bekannt, dass man substituierte 6H-1,2,4,6-Thiatriazin(5)on-1,1-dioxidderivate erhält, wenn man beispielsweise Addukte aus 2 Mol Isocyanat und einem Mol Chlorsulfonylisocyanat mit Alkoholen umsetzt (DOS 1 946 262), oder wenn man N′-Carboalkoxy-N-sulfamoyl-guanidine unter alkalischen Bedingungen cyclisiert (DOS 2 508 832). Dort wird auch ihre herbizide Wirkung beschrieben.

Es wurde gefunden, dass 6H-1,2,4,6-thiatriazin-5-halogen-1,1-dioxide der allgemeinen Formel I

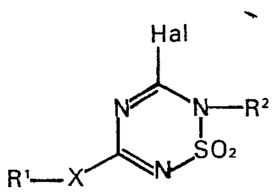

in der

$R^1$ einen gesättigten geradkettigen aliphatischen Rest mit 1 bis 10 Kohlenstoffatomen, einen cycloaliphatischen Rest mit 3 bis 7 Kohlenstoffatomen, einen verzweigten, gesättigten oder ungesättigten aliphatischen Rest mit 3 bis 10 Kohlenstoffatomen, einen halogen-, alkoxy- oder alkylmercapto-substituierten geradkettigen oder verzweigten aliphatischen Rest mit 2 bis 10 Kohlenstoffatomen, einen cycloalkyloxy-substituierten aliphatischen Rest mit 4 bis 10 Kohlenstoffatomen, einen gegebenenfalls halogen-, niedrigalkyl-, niedrigalkoxy-substituierten Phenylrest oder einen gegebenenfalls halogen-substituierten Benzylrest, Allyl, Methallyl, Crotyl, 2-Ethylhexen-2-yl-1, Hexen-5-yl-1, 2-Methyl-buten-2-yl-1, 2-Methyl-buten-1-yl-3, Butin-1-yl-3, Butin-2-yl-1, Buten-1-yl-3, Propargyl, 2-Methyl-buten-1-yl-4, 2-Methyl-buten-2-yl-4, 3-Methyl-buten-1-yl-3, 1-Cyclohexlethyl,

$R^2$ Wasserstoff, einen geradkettigen aliphatischen Rest mit 1 bis 10 Kohlenstoffatomen, einen cycloaliphatischen Rest mit 3 bis 7 Kohlenstoffatomen, einen verzweigten aliphatischen Rest mit 3 bis 10 Kohlenstoffatomen oder einen halogen- oder alkoxy-substituierten Alkylrest mit 2 bis 10 Kohlenstoffatomen,

X Sauerstoff, Schwefel, eine Sulfinyl- oder Sulfonylgruppe und der Rest Hal ein Halogenatom bedeutet, eine gute herbizide Wirkung haben und wertvolle Zwischenprodukte zur Herstellung von Wirkstoffen für Pflanzenschutzmittel, Farbstoffe und Pharmazeutika sind.

In der Formel I stehen $R^1$ und $R^2$ beispielsweise für Methyl, Ethyl, Propyl, Isopropyl, Cyclopropyl, Butyl, Isobutyl, sek.-Butyl, tert.-Butyl, 1-Pentyl, Cyclopentyl, Hexyl, Cyclohexyl, 3-Pentyl, 1,2-Dimethylpropyl, 1,3-Dimethylbutyl, 2-Chlorethyl, 2-Chlorpropyl, 3-Chlorpropyl, 2-Chlorisopropyl, 1-Chlormethylpropyl, 1-Ethyl-2-methylpropyl, 1,2,2-Trimethylpropyl, 1,2-Dimethylhexyl, 1-Cyclohexylethyl, 2-Chlorbutyl-3, 2-Chlor-2-methylpropyl, 2-Fluorbutyl-3, 2-Fluor-2-methylpropyl, 2-Fluorisopropyl, tert.-Amyl, Chlor-tert.-butyl, 2,2,2-Trifluorethyl, Methoxyethyl, Ethoxyethyl, 3-Methoxypropyl, Methoxyisopropyl, 3-Methoxybutyl, 1-Methoxy-butyl-2, Ethoxy-tert.-butyl, Methoxy-tert.-butyl, 2-Methoxy-butyl, 4-Methoxy-butyl.

Ferner kann $R^1$ beispielsweise Allyl, Methallyl, Crotyl, 2-Ethyl-hexen-2-yl-1, Hexen-5-yl-1, 2-Methyl-buten-2-yl-1, 2-Methyl-buten-1-yl-3, Butin-1-yl-3, Butin-2-yl-1, Buten-1-yl-3, Propargyl, 2-Methyl-buten-1-yl-4, 2-Methyl-buten-2-yl-4, 3-Methyl-buten-1-yl-3, Methylmercapto-ethyl, Ethylmercapto-ethyl, 3-Methylmercapto-propyl, 3-Methylmercapto-butyl, 1-Methylmercapto-butyl-2, Methylmercapto-tert.-butyl, 2-Methylmercapto-butyl, Cyclohexoxy-ethyl, Benzyl, 2,6-Dichlorbenzyl, 2-Chlor-6-fluorbenzyl, 2,6-Difluorbenzyl, Phenyl, 4-Chlorphenyl, 3,4-Dichlorphenyl, 4-tert.-Butylphenyl, 4-Methoxy-3-chlorphenyl, 4-Methoxyphenyl, 2-Methylphenyl oder 2-Methyl-4-chlorphenyl bedeuten.

Die Bezeichnung Hal bedeutet Fluor, Chlor, Brom oder Jod.

Es wurde ferner gefunden, dass man die neuen Verbindungen in einfacher Weise erhält, wenn man Verbindungen der allgemeinen Formel II oder deren Alkali- oder Erdalkalisalze

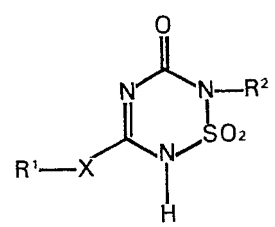

II

worin $R^1$, $R^2$ und X die vorgenannte Bedeutung besitzen, mit einem Säurehalogenid der Phosphorsäure, phosphorigen Säure, Kohlensäure, Oxalsäure oder schwefligen Säure gegebenenfalls in Gegenwart eines Lösungs- oder Verdünnungsmittels und gegebenenfalls in Gegenwart eines Reaktionsbeschleunigers im allgemeinen bei Temperaturen zwischen 0 und 160°C, drucklos oder unter Druck, kontinuierlich oder diskontinuierlich umsetzt.

Verwendet man 6-Ethyl-3-methoxy-6H-1,2,4,6-thiatriazin(5)-on-1,1-dioxid und Phosgen als Ausgangsmaterialien, so kann der Reaktionsablauf durch folgendes Formelschema wiedergegeben werden:

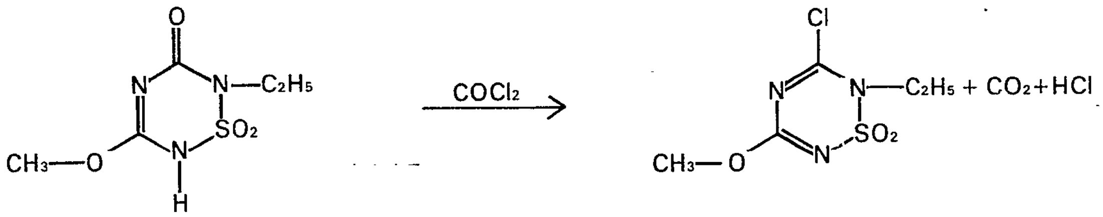


Zweckmässig verwendet man für die Umsetzung unter den Reaktionsbedingungen inerte Lösungs- oder Verdünnungsmittel. Als Lösungsmittel kommen z.B. in Frage:

Halogenkohlenwasserstoffe, insbesondere Chlorkohlenwasserstoffe, z.B. Tetrachlorethylen, 1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Tetrachlorethan, Trichlorethylen, Pentachlorethan, δ-, m-, p-Difluorbenzol, 1,2-Dichlorethan, 1,1-Dichlorethan, 1,2-cis-Dichlorethylen, Chlorbenzol, Fluorbenzol, Brombenzol, o-, m-, p-Dichlorbenzol, o-, m-, p-Dibrombenzol, o-, m-, p-Chlor-toluol, 1,2,4-Trichlorbenzol oder Ether, z.B. Ethylpropylether, Methyl-tert.-butylether, n-Butylether, Diisopropylether, Anisol, Dioxan, Ethylenglykoldimethylether oder Nitro-kohlenwasserstoffe, z.B. Nitrobenzol, o-, m-, p-Chlornitrobenzol, o-Nitrotoluol oder Nitrile z.B. Acetonitril, Butyronitril, Isobutyronitril, Benzonitril, m-Chlorbenzonitril oder aliphatische oder cycloaliphatische Kohlenwasserstoffe, z.B. Heptan, Pinan, Nonan, o-, m-, p-Cymol, Benzinfraktionen innerhalb eines Siedepunktintervalles von 70 bis 190°C, Cyclohexan, Methylcyclohexan, Dekalin, Petrolether, Hexan, Ligroin, 2,2,4-Trimethylpentan, 2,2,3-Trimethylpentan oder Ester z.B. Ethylacetat und entsprechende Gemische. Bevorzugte Lösungsmittel sind ferner anorganische Säurechloride, z.B. Phosphoroxychlorid oder entsprechende Mischungen mit inerten Chlorkohlenwasserstoffen wie beispielsweise 1,2-Dichlorethan. Zweckmässig verwendet man das Lösungsmittel in einer Menge von 100 bis 2000 Gewichtsprozent, vorzugsweise von 200 bis 700 Gewichtsprozent, bezogen auf den Ausgangsstoff der Formel II.

Bevorzugte Säurehalogenide sind Thionylchlorid, Schwefeltetrafluorid, Phosgen, Oxalsäurechlorid, Phosphortribromid und insbesondere Phosphorpentachlorid, Phosphortrichlorid und Phosphoroxychlorid. Die Umsetzung wird im allgemeinen mit einer Menge von 1,0 bis 1,5, vorzugsweise 1,05 bis 1,2 Mol Säurehalogenid bezogen auf Ausgangsstoff II durchgeführt. Im Falle der Verwendung fünfwertiger Phosphorhalogenverbindungen verwendet man 0,7 bis 1,5, vorzugsweise 1,0 bis 1,2 Mol Phosphorpentahalogenid bezogen auf Ausgangsstoff II.

Im Falle der Verwendung eines Phosphor(V)halogenids als Halogenierungsagens empfiehlt sich die Verwendung eines Phosphoroxyhalogenids als Verdünnungsmittel, das zweckmässig in einer Menge von 1 bis 10 Mol Phosphoroxyhalogenid, bezogen auf Ausgangsstoff II eingesetzt wird.

Hierbei kann das Phosphor(V)halogenid auch direkt in situ hergestellt werden, indem man beispielsweise eine Mischung eines Phosphor-IIIhalogenids in Phosphoroxyhalogenid oder einem der vorgenannten inerten Lösungsmittel mit der erforderlichen stöchiometrischen Menge aktivem Halogen umsetzt, beispielsweise nach dem in der US-Patentschrift 1 906 440 beschriebenen Verfahren und dann nach Zugabe des Ausgangsstoffes II die Umsetzung durchführt.

Als Reaktionsbeschleuniger kann man vorteilhaft ein am Stickstoffatom disubstituiertes, gegebenenfalls cyclisches Carbonsäureamid, einen tetraalkylsubstituierten Harnstoff, ein tertiäres Amin, vorteilhaft in einer Menge von 1 bis 10 Gewichtsprozent, bezogen auf den Ausgangsstoff II, verwenden. Auch Gemische der genannten Katalysatoren kommen für die Reaktion in Betracht. Ferner kann man auch Salze von Diaminen, z.B. die Hydrochloride der Amine, oder ein quaternäres Salz eines Amins verwenden. Bevorzugte Katalysatoren sind:

Triethylamin, Pyridin, N,N-Dimethylanilin, N-Ethylpiperidin, N-Methylpyrrolidin, α, β oder γ-Picolin, Chinolin, Isochinolin, Chinazolin, Chinoxalin, N-Propyldiisopropylamin, 2,6- und 2,4-Lutidin, N-(4-Pyridyl)-pyridiniumchlorid-hydrochlorid, p-Dimethylaminopyridin, Pyrimidin, Acridin, Dimethylformamid, Diethylformamid, Ameisensäure-N-methylanilid, N,N-Dimethylacetamid, N-Methylpyrrolidon, Tetramethylharnstoff.

Die als Ausgangsstoffe II benötigten 6H-1,2,4,6-Thiatriazin(5)-on-1,1-dioxide sind teilweise bekannt oder lassen sich durch Umsetzung von N-Carbo-alkoxy-O-alkyl-iso Harnstoffen mit Aminosulfonylhalogeniden herstellen. Nach ihren spektroskopischen Daten liegen sie in der unter der Formel II wiedergegebenen Strukturform vor. Je nach Lösungsmittel können dabei jedoch auch gewisse Anteile der tautomeren Form IIa auftreten, die als im Gleichgewicht befindliche Verbindungen gleichermassen bevorzugte Ausgangsmaterialien darstellen.

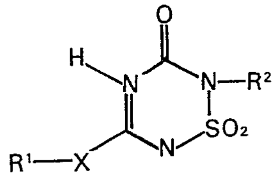

IIa

Zweckmässigerweise wird das Verfahren zur Herstellung der neuen Verbindungen so durchgeführt, dass man den Ausgangsstoff II, gegebenenfalls zusammen mit einem der vorgenannten inerten Verdünnungsmittel vorlegt, das Halogenierungsagens bei 0 bis 60°C, vorzugsweise bei 20 bis 40°C zugibt und dann je nach Massgabe der Gasabspaltung erhitzt.

Man kann jedoch auch den Ausgangsstoff II, gegebenenfalls in einem der vorgenannten inerten Verdünnungsmittel zu dem Halogenierungsagens zugeben. Im Falle des Phosgens empfiehlt sich die Zugabe eines Reaktionsbeschleunigers.

Zur Beendigung der Umsetzung rührt man noch 0,5 bis 15 Stunden bei 0 bis 160°C, vorzugsweise bei 80 bis 130°C nach. Der Umsetzungsgrad lässt sich hierbei in einfacher Weise durch spektroskopische Methoden, beispielsweise durch die Verschiebung der Protonenresonanzsignale der Reste $R^2$ oder $R^1$ verfolgen.

Aus dem Reaktionsgemisch wird der Endstoff I in üblicher Weise, z.B. nach Abdestillieren von Lösungsmittel und überschüssigem Halogenierungsagens, isoliert. Die gewünschten Endstoffe fallen hierbei in reiner Form an, gegebenenfalls können sie durch Umkristallisation, Chromatographie oder Destillation gereinigt werden. Die folgenden Vorschriften betreffen die Herstellung der Ausgangsverbindungen der Formel II.

Vorschrift 1

198 Teile (Gewichtsteile) Methylaminosulfonylchlorid und 162 Teile Triäthylamin wurden gleichzeitig über 2 Zuführungen bei 25 bis 30° C unter Rühren in eine Mischung von 202 Teilen N-Carbomethoxy-O-methylisoharnstoff in 1570 Teilen Acetonitril eingeführt. Nach 3 Stunden Rühren bei 25°C wurde vom ausgefallenen Hydrochlorid abgesaugt und das Filtrat im Vakuum eingeengt. Der Rückstand wurde in 1500 Teilen 1,2-Dichlorethan gelöst, einmal mit Wasser und zweimal mit 0,5 normaler Salzsäure extrahiert. Nach dem Trocknen über Magnesiumsulfat und Einengen im Vakuum wurden 257 Teile N-Carbomethoxy-N'-methylsulfamoyl-O-methylharnstoff mit $n_D^{25}$ 1,4851 erhalten.

Hiervon wurden 96 Teile in 235 Teilen Methanol (absolut trocken) gelöst, mit 153,5 Teilen Natriummethylat (30 Gewichtsprozent) versetzt und 3 Stunden unter Rückfluss gerührt. Nach dem Einengen im Vakuum wurde der Rückstand in Wasser gelöst, einmal mit Äther extrahiert und mit verdünnter Schwefelsäure angesäuert. Nach dem Absaugen, Waschen mit Wasser und Trocknen wurden 68 Teile (= 82,5% der Theorie) 6-Methyl-3-methoxy-6H-1,2,4,6-thiatriazin(5)-on-1,1-dioxid mit Fp. 198 bis 202 °C erhalten.

Vorschrift 2

37,9 Teile Isopropylaminosulfonylchlorid und 26,3 Teile Tritäyhlamin wurden über zwei Zuführungen gleichzeitig zu einer Mischung von 54 Teilen N-Carbomethoxy-S-benzyl-isothioharnstoff in 740 Teilen 1,2-Dichlorethan unter Rühren bei 5 bis 10°C zugegeben. Nach 4 Stunden Rühren bei 25°C wurde das Reaktionsgemisch einmal mit 200 Teilen Wasser und zweimal mit je 100 Teilen 0,5 normaler Salzsäure extrahiert. Nach dem Trocknen und Einengen im Vakuum erhielt man 79 Teile N-Carbomethoxy-N'-isopropylsulfamoyl-S-benzyl-isothioharnstoff mit $n_D^{25}$ 1,5598. Dieser kristallisierte beim Anreiben mit Hexan mit Fp. 76 bis 78°C. 76 Teile N-Carbomethoxy-N'-isopropylsulfamoyl-S-benzylisothioharnstoff wurden in 244 Teilen einer Mischung von 44 Teilen Natronlauge (50 Gewichtsprozent) und 200 Teilen Wasser gelöst und 5 Minuten bei 85°C gerührt. Die Reaktionsmischung wurde abgekühlt, mit 15%iger Salzsäure angesäuert und das ausgefallene Öl in Methylenchlorid aufgenommen. Nach dem Trocknen über Magnesiumsulfat, Filtrieren über neutralem Aluminiumoxid und Einengen in Vakuum wurden 59,5 Teile 6-Isopropyl-3-benzylmercapto-6H-1,2,4,6-thiatriazin(5)on-1,1-dioxid (86,3% der Theorie) mit Fp. 124 bis 130°C erhalten.

Vorschrift 3

59,6 Teile Cyclohexylaminosulfonylchlorid und 26,9 Teile Pyridin wurden über zwei Zuführungen bei 15 bis 20°C unter Rühren in eine Lösung von 39,6 Teilen N-Carbomethoxy-O-methylisoharnstoff in 300 Teilen Essigester eingeführt. Nach 4 Stunden Rühren bei 25°C wurde das Reaktionsgemisch je einmal mit Wasser und mit 0,5 normaler Salzsäure extrahiert, getrocknet und dann im Vakuum eingeengt. Hierbei wurden 79 Teile N-Carbomethoxy-N'-cyclohexylsulfamoyl-O-methylisoharnstoff mit $n_D^{25}$ 1,4970 erhalten. Nach dem Anreiben mit wenig Äther kristallisierte die Verbindung mit Fp. 84 bis 86°C. 15 Teile N-Carbomethoxy-N'-cyclohexylsulfamoyl-O-methylisoharnstoff wurden in einer Mischung von 9 Teilen Natronlauge (50 Gewichtsprozent) und 20 Teilen Wasser gelöst und 4 Minuten bei 55 bis 60°C gerührt. Nach dem Abkühlen wurde die Reaktionslösung einmal mit Ether extrahiert und dann in eine Mischung von 9,5 Teilen konzentrierte Salzsäure in 10 Teilen Wasser eingerührt. Nach dem Absaugen, Waschen mit Wasser und Trocknen wurden 9 Teile 6-Cyclohexyl-3-methoxy-6H-1,2,4,6-thiatriazin(5)on-1,1-dioxid mit Fp. 173 bis 177°C erhalten.

Vorschrift 4

95 Teile Isopropylaminosulfonylchlorid wurden bei 10 bis 15°C innerhalb 25 Minuten in eine Mischung von 96 Teilen N-Carbomethoxy-O-isopropylisoharnstoff und 73 Teilen Triäthylamin in 700 Teilen Tetrahydrofuran eingerührt. Nach einer Stunde Rühren bei 25°C wurde das Reaktionsgemisch je einmal mit Wasser und mit 0,5 normaler Salzsäure extrahiert, getrocknet und im Vakuum eingeengt. Dabei wurden 130 Teile N-Carbomethoxy-N'-isopropylsulfamoyl-O-isopropylisoharnstoff mit Fp. 62 bis 64°C erhalten. Hiervon wurden 33,7 Teile mit 17,6 Teilen Natronlauge (50 Gewichtsprozent) in 30 Teilen Wasser während 5 Minuten bei 55 bis 60°C cyclisiert. Nach dem Extrahieren mit Ether, Ansäuern, Waschen mit Wasser und Trocknen wurden 22 Teile 6-Isopropyl-3-isopropoxy-6H-1,2,4,6-thiatriazin(5)on-1,1-dioxid mit Fp. 164 bis 167°C erhalten.

Vorschrift 5

12 Teile 6-Isopropyl-3-isopropoxy-6H-1,2,4-thiatriazin(5)on-1,1-dioxid wurden in einer Mischung von 10,4 Teilen Natriummethylat (30 Gewichtsprozent) und 64 Teilen Methanol bei 25°C gelöst. Nach dem Einengen wurden 13,8 Teile 6-Isopropyl-3-isopropoxy-6H-1,2,4,6-thiatriazin(5)on-1,1-dioxid-2-Natriumsalz mit Fp. 123°C Zersetzung erhalten.

Vorschrift 6

140 Teile N-Carboxymethyl-N′-methylsulfamoyl-O-methylharnstoff wurden in einer Mischung von 79,5 Teilen Natriumcarbonat in 450 Teilen Wasser und 31 Volumenteilen 2 n Natronlauge 10 Minuten bei 45°C gerührt. Das Reaktionsgemisch wurde abgekühlt, mit Äther extrahiert und langsam in eine Mischung von 78 Teilen konzentrierte Schwefelsäure in 150 Teilen Eiswasser eingerührt. Nach dem Absaugen, Waschen mit Wasser und Trocknen wurden 81 Teile 6-Methyl-3-methoxy-6H-1,2,4,6-thiatriazin(5)on-1,1-dioxid (68% der Theorie) mit Fp. 195 bis 199°C erhalten.

Die folgenden Beispiele betreffen die Herstellung der neuen Stoffe.

Beispiel 1

215 Teile 6-Methyl-3-methoxy-6H-1,2,4,6-thiatriazin(5)on-1,1-dioxid werden bei Raumtemperatur in eine Mischung von 275 Teilen Phosphorpentachlorid in 1480 Teilen Phosphoroxychlorid unter Rühren eingeführt und innerhalb 30 Minuten auf 110°C erhitzt. Nach 4 Stunden Rühren unter Rückfluss wurde das Reaktionsgemisch im Vakuum eingeengt, wobei 235 Teile (99,6% der Theorie) 5-Chlor-6-methyl-3-methoxy-6H-1,2,4,6-thiatriazin-1,1-dioxid mit Fp. 77–83°C erhalten wurden (Verbindung Nr. 1).

Beispiel 2

154 Teile Phosphorpentachlorid wurden innerhalb 2 Minuten unter Rühren bei 25°C zu einer Mischung von 128 Teilen 6-Ethyl-3-methoxy-6H-1,2,4,6-thiatriazin(5)on-1,1-dioxid in 840 Teilen Phosphoroxychlorid gegeben. Das Reaktionsgemisch wurde 5½ Stunden unter Rückfluss gerührt und dann im Vakuum eingeengt. Das verbleibende Öl wurde in 300 Teilen 1,2-Dichlorethan aufgenommen und über neutrales Aluminiumoxid (Aktivität I) chromatographiert. Nach dem Einengen wurden 127,5 Teile (91% d. Theorie) 5-Chlor-6-ethyl-3-methoxy-6H-1,2,4,6-thiatriazin-1,1-dioxid mit Fp. 63–70°C erhalten (Verbindung Nr. 2).

Beispiel 3

50 Teile Phosphorpentachlorid wurden unter Rühren bei Raumtemperatur zu einer Mischung von 41,4 Teilen 6-Methyl-3-ethoxy-6H-1,2,4,6-thiatriazin(5)on-1,1-dioxid in 100 Teilen 1,2-Dichlorethan und 100 Teilen Phosphoroxyhalogenid gegeben. Die Reaktionsmischung wurde 12 Stunden unter Rückfluss gerührt. Nach dem Einengen im Vakuum wurden 43,5 Teile (96,5% d. Theorie) 5-Chlor-6-methyl-3-ethoxy-6H-1,2,4,6-thiatriazin-1,1-dioxid mit Fp. 75–80°C erhalten (Verbindung Nr. 3).

Beispiel 4

50 Teile Phosphorpentachlorid wurden zu einer Mischung von 44 Teilen 6-n-Propyl-3-methoxy-6H-1,2,4,6-thiatriazin(5)on-1,1-dioxid und 268 Teilen Phosphoroxychlorid unter Rühren bei 22°C gegeben und innerhalb 20 Minuten auf 110°C erhitzt. Nach 7 Stunden Rühren unter Rückfluss wurde die Reaktionsmischung im Vakuum eingeengt, wobei 45 Teile öliges 5-Chlor-6-n-propyl-3-methoxy-6H-1,2,4,6-thiatriazin-1,1-dioxid erhalten wurden; NMR: ($CDCl_3$) N-$CH_2$ 4.0–4.28 δ.

Durch Destillation bei 125–130°C/0,01 mbar wurden 40,3 Teile (84% der Theorie) Reinprodukt erhalten (Verbindung Nr. 4).

Beispiel 5

Eine Suspension von 25 Teilen 6-Ethyl-3-methylmercapto-6H-1,2,4,6-thiatriazin(5)on-1,1-dioxid in 2,5 Teilen DMF und 245 Teilen 1,2-Dichlorethan wurden während 14 Stunden unter Rühren bei 83°C mit Phosgen begast. Nach dem Einengen im Vakuum wurden 27 Teile viskoses Öl isoliert, das nach dem NMR-Spektrum ca. 45% 5-Chlor-6-ethyl-3-methylmercapto-6H-1,2,4,6-thiatriazin-1,1-dioxid enthielt. Eine Probe wurde bei 136–144°C/0,01 mbar destilliert; NMR: ($CDCl_3$) N-$CH_2$ 4.04–4.42 δ (q), $CH_3S$ 2.52 δ (s) (Verbindung Nr. 5).

Weitere Beispiele für neue Verbindungen sind nachfolgend in Tabelle 1 aufgeführt:

Tabelle 1

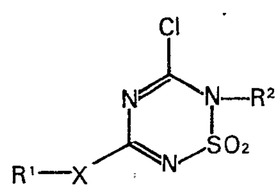

| Verbindung Nr. | $R^1$ | $R^2$ | X | Fp. [°C] oder $n_D^{25}$ |
|---|---|---|---|---|
| 6 | $CH_3$ | i-$C_3H_7$ | O | 1,5237 |
| 7 | $CH_3$ | sec-$C_4H_9$ | O | |
| 8 | $CH_3$ | i-$C_4H_9$ | O | |
| 9 | $CH_3$ | tert.-$C_4H_9$ | O | |

| Verbindung Nr. | $R^1$ | $R^2$ | X | Fp. [°C] oder $n_D^{25}$ |
|---|---|---|---|---|
| 10 | $CH_3$ | 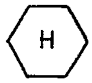 | O | 105-109 |
| 11 | $CH_3$ | $CH_2$-$CH_2Cl$ | O | |
| 12 | $CH_3$ | $CH_2$-$CH_2$-O-$CH_3$ | O | |
| 13 | $C_2H_5$ | $C_2H_5$ | O | 1,5150 |
| 14 | $C_2H_5$ | n-$C_3H_7$ | O | 1,5100, Sdp. 118/0.01 |
| 15 | $C_2H_5$ | i-$C_3H_7$ | O | 1,5201, Sdp. 119-121/0.01 |
| 16 | $C_2H_5$ | n-$C_4H_9$ | O | |
| 17 | $C_2H_5$ | i-$C_4H_9$ | O | |
| 18 | $C_2H_5$ | sec-$C_4H_9$ | O | |
| 19 | $C_2H_5$ | tert.-$C_4H_9$ | O | |
| 20 | $C_2H_5$ | $CH_2$-$CH_2Cl$ | O | |
| 21 | $C_2H_5$ | 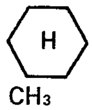 | O | |
| 22 | n-$C_3H_7$ | $CH_3$ | O | 37-41 |
| 23 | n-$C_3H_7$ | $C_2H_5$ | O | |
| 24 | n-$C_3H_7$ | $C_2H_5$ | S | |
| 25 | n-$C_3H_7$ | n-$C_3H_7$ | O | |
| 26 | n-$C_3H_7$ | i-$C_3H_7$ | O | |
| 27 | n-$C_3H_7$ | sec-$C_4H_9$ | O | |
| 28 | n-$C_3H_7$ | tert.-$C_4H_9$ | O | |
| 29 | n-$C_3H_7$ | $CH_2CH_2$-O-$CH_3$ | O | |
| 30 | n-$C_3H_7$ | 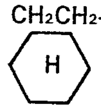 | O | |
| 31 | i-$C_3H_7$ | $CH_3$ | O | viskos, NMR ($CDCl_3$) H-C-O 4.97-5,3δ |
| 32 | i-$C_3H_7$ | $C_2H_5$ | O | |
| 33 | i-$C_3H_7$ | n-$C_3H_7$ | O | 1,5009 |
| 34 | i-$C_3H_7$ | i-$C_3H_7$ | O | viskos, NMR ($CDCl_3$) H-C 4.9-5.4δ, $CH_3$-C 1.35, 1.45, 1.62, 1.72δ |
| 35 | i-$C_3H_7$ | sec.-$C_4H_9$ | O | |
| 36 | i-$C_3H_7$ | 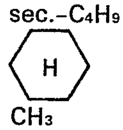 | O | |
| 37 | n-$C_4H_9$ | $CH_3$ | O | |
| 38 | n-$C_4H_9$ | $C_2H_5$ | O | |
| 39 | n-$C_4H_9$ | n-$C_3H_7$ | O | |
| 40 | n-$C_4H_9$ | i-$C_3H_7$ | O | |
| 41 | n-$C_4H_9$ | sec.-$C_4H_9$ | O | |
| 42 | n-$C_4H_9$ | $CH_2$-$CH_2Cl$ | O | |
| 43 | n-$C_4H_9$ | i-$C_3H_7$ | S | |
| 44 | i-$C_4H_9$ | $CH_3$ | O | |
| 45 | i-$C_4H_9$ | i-$C_3H_7$ | O | 1,5049 |
| 46 | sec.-$C_4H_9$ | $CH_3$ | O | |
| 47 | sec.-$C_4H_9$ | $C_2H_5$ | O | |
| 48 | sec.-$C_4H_9$ | i-$C_3H_7$ | O | viskos, NMR ($CDCl_3$) $(CH_3)_2C$ 1,66, 1,77δ |
| 49 | tert.-$C_4H_9$ | $CH_3$ | O | |
| 50 | tert.-$C_4H_9$ | $C_2H_5$ | O | |
| 51 | tert.-$C_4H_9$ | i-$C_3H_7$ | O | |
| 52 | 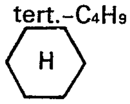 | $CH_3$ | O | 45-53 |
| 53 | 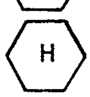 | $C_2H_5$ | O | |
| 54 | 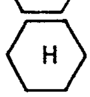 | i-$C_3H_7$ | O | |

| Verbindung Nr. | $R^1$ | $R^2$ | X | Fp. [°C] oder $n_D^{25}$ |
|---|---|---|---|---|
| 55 | $CH_2{=}CH{-}CH_2$ | $CH_3$ | O | |
| 56 | $CH_2{=}CH{-}CH_2$ | $C_2H_5$ | O | |
| 57 | $CH_2{=}CH{-}CH_2$ | $i{-}C_3H_7$ | O | |
| 58 | $C_6H_5{-}CH_2$ | $CH_3$ | O | |
| 59 | $C_6H_5{-}CH_2$ | $i{-}C_3H_7$ | O | |
| 60 | 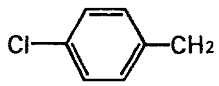 | $CH_3$ | O | |
| 61 | $CH_3{-}O{-}CH_2{-}CH_2$ | $CH_3$ | O | |
| 62 | $CH_3{-}O{-}CH_2{-}CH_2$ | $C_2H_5$ | O | |
| 63 | $CH_3{-}S{-}CH_2{-}CH_2$ | $CH_3$ | O | |
| 64 | $CH_3{-}S{-}CH_2{-}CH_2$ | $i{-}C_3H_7$ | O | |
| 65 | $CH_3$ | $CH_3$ | S | 103–107 |
| 66 | $C_2H_5$ | $CH_3$ | S | |
| 67 | $n{-}C_3H_7$ | $CH_3$ | S | |
| 68 | $CH_3$ | $i{-}C_3H_7$ | S | |
| 69 | $CH_3$ | $n{-}C_3H_7$ | S | |
| 70 | $C_6H_5{-}CH_2$ | $CH_3$ | S | viskos, NMR ($CDCl_3$) $CH_3$ 3.4δ, $CH_2$ 4.47δ |
| 71 | 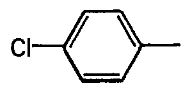 | $i{-}C_3H_7$ | S | 146–148 |
| 72 | 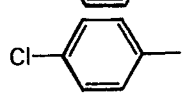 | $CH_3$ | S | 109 |

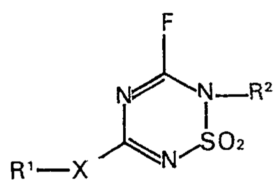

| Verbindung Nr. | $R^1$ | $R^2$ | X | Fp. [°C] oder $n_D^{25}$ |
|---|---|---|---|---|
| 73 | $CH_3$ | $CH_3$ | O | viskos, NMR ($d_6$DMSO) O–$CH_3$ 3.9δ, N–$CH_3$ 3.1δ |
| 74 | $CH_3$ | $C_2H_5$ | O | |
| 75 | $C_2H_5$ | $CH_3$ | O | |
| 76 | $CH_3$ | $CH_3$ | S | |
| 77 | $C_2H_5$ | $CH_3$ | S | |

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwekken; sie sollen in jedem Fall eine feine und gleichmässige Verteilung der wirksamen Substanz gewährleisten. Die Formulierungen werden in bekannter Weise hergestellt, z.B. durch Verstrekken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ehtanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen - Fettalkohol - Ether, Alkylsulfonate und Arylsulfonate) und Dispergiermittel, wie Lignin, Sulfitablaugen und Methylcellulose.

Die herbiziden Mittel enthalten im allgemeinen zwischen 0,1 und 95 Gew.-% Wirkstoff, vorzugsweise zwischen 0,5 und 90.

Die Aufwandmengen liegen je nach Zusammensetzung und Wachstumsstadien der Unkrautflora zwischen 0,1 und 15, vorzugsweise jedoch zwischen 0,2 und 5 kg Wirkstoff pro Hektar, wobei für eine totale Pflanzenvernichtung die höheren Aufwandmengen zu verwenden sind.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Giessen.

Beispiele für solche Zubereitungen sind:

Beispiel I

Man vermischt 90 Gewichtsteile der Verbindung 1 mit 10 Gewichtsteilen N-Methyl-alphapyrrolidon und erhält eine Lösung, die zur Anwendung in Form kleinster Tropfen geeignet ist.

Beispiel II

10 Gewichtsteile der Verbindung 2 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-mono-ethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht.

Beispiel III

20 Gewichtsteile der Verbindung 2 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

Beispiel IV

20 Gewichtsteile der Verbindung 3 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht.

Beispiel V

80 Gewichtsteile des Wirkstoffs 1 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

Beispiel VI

5 Gewichtsteile der Verbindung 1 werden mit 95 Gewichtsteilen feinteiligem Kaolin innig vermischt. Man erhält auf diese Weise ein Stäubemittel, das 95 Gewichtsprozent des Wirkstoffs enthält.

Beispiel VII

30 Gewichtsprozent der Verbindung 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel VIII

40 Gewichtsteile des Wirkstoffs 1 werden mit 10 Teilen Natriumsalz eines Phenolsulfonsäure-harnstoff-formaldehyd-kondensats, 2 Teilen Kieselgel und 48 Teilen Wasser innig vermischt. Man erhält eine stabile wässrige Dispersion.

Beispiel IX

20 Teile des Wirkstoffs 1 werden mit 12 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-kondensats und 68 Teilen eines paraffinischen Mineralöls innig vermischt. Man erhält eine stabile ölige Dispersion.

Die neuen Verbindungen können unter sich und mit anderen Herbiziden sowie auch noch mit weiteren Pflanzenschutzmitteln gemischt und gemeinsam ausgebracht werden, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden.

Die herbizide Aktivität der neuen Verbindungen und ihre Verträglichkeit für gewisse Kulturpflanzen wird in einigen Gewächshausversuchen gezeigt.

Versuch 1

In Plastikeimer von 10 Liter Fassungsvermögen wurde Lehmboden eingefüllt. Diesen besetzte man mit Knollen von Cyperus esculentus. Ferner säte man Samen von Cyperus iria dazwischen. Nach einigen Tagen wurden junge Reispflanzen von etwa 10 cm Sprosslänge eingepflanzt. Der Lehmboden wurde schon von Anfang an schlammig nass gehalten. Nach dem Einpflanzen der Reispflanzen wurde Wasser 3 cm hoch über die bodenoberfläche angestaut. In dieses so simulierte Miniaturreisfeld streute oder spritzte man die herbiziden Wirkstoffe unter Beachtung einer möglichst gleichmässigen Verteilung. Hierbei bewirkte 5-chlor-6-methyl-3-methoxy-6H-1,2,4,6-thiatriazin-1,1-dioxid in Aufwandmengen von 1,0 und 2,0 kg/ha eine ganz beachtliche Unterdrükkung der Cyperaceen. Die Reispflanzen wurden nicht oder nur unwesentlich und temporär geschädigt.

Versuch 2

In Plastikblumentöpfe von 300 $cm^3$ Inhalt wurden die Samen der Testpflanzen entsprechend Tabelle 1, nach Arten getrennt, in lehmigen Sandboden flach eingesät. Unmittelbar nach der Einsaat erfolgte bei Vorauflaufbehandlung das Aufbringen der Wirkstoffe auf die Erdoberfläche. Anschliessend wurden die Gefässe leicht beregnet, um Keimung und Wachstum in Gang zu bringen und auch gleichzeitig die Einwirkung der Wirkstoffe zu ermöglichen. Danach deckte man die Gefässe mit durchsichtigen Plastikhauben ab, bis die Pflanzen angewachsen waren. Diese Abdekkung bewirkte ein gleichmässiges Keimen der Testpflanzen, sofern dies nicht durch die Wirkstoffe beeinträchtigt wurde und verhinderte das Verdampfen leicht flüchtiger Substanzen.

Zum Zwecke der Nachauflaufbehandlung zog man die Pflanzen je nach Wuchsform in den Versuchsgefässen erst bis zu einer Höhe von 3 bis 10 cm an und behandelte sie danach. Eine Abdekkung unterblieb. Die Aufstellung der Versuche erfolgte im Gewächshaus, wobei für wärmeliebende Arten wärmere Bereiche (25 bis 40°C) und für solche gemässigter Klimate 15 bis 30°C bevorzugt wurden. Die Versuchsperiode erstreckte sich über 3–5 Wochen. Während dieser Zeit wurden die Pflanzen gepflegt und ihre Reaktion auf die einzelnen Behandlungen wurde ausgewertet. Die folgenden Tabellen enthalten die Prüfsubstanzen, die jeweiligen Dosierungen in kg/ha Aktivsubstanz und die Testpflanzenarten. Bewertet wird nach einer Skala von 0 bis 100. Dabei bedeutet 0 keine Schädigung oder normales Wachstum und 100 kein Aufgang der Pflanzen bzw. völlige Zerstörung zumindest der oberirdischen Sprossteile.

Die beigefügten Tabellen zeigen nochmals die bereits oben erwähnte, selektive herbizide Wirkung von 5-Chlor-6-methyl-3-methoxy-6H-1,2,4,6-thiatriazin-1,1-dioxid bei Vor- und Nachauflaufanwendung. Eine besondere Ausbringungstechnik besteht darin, dass die Wirkstoffe mit Hilfe der Spritzgeräte so gespritzt werden, dass die Blätter empfindlicher Kulturpflanzen nach Möglichkeit nicht getroffen werden, während die Wirkstoffe auf die darunterliegende Bodenfläche oder dort wachsende unerwünschte Pflanzen gelangen (post-directed, lay-by).

In Anbetracht der Vielseitigkeit der Applikationsmethoden können die erfindungsgemässen Mittel oder diese enthaltende Mischungen noch in einer grossen Zahl von Kulturen zur Beseitigung unerwünschten Pflanzenwuchses eingesetzt werden.

Im einzelnen seien folgende Nutzpflanzen genannt:

| Botanischer Name | Deutscher Name | Englischer Name |
|---|---|---|
| Allium cepa | Küchenzwiebel | onions |
| Ananas comosus | Ananas | pineapple |
| Arachis hypogaea | Erdnuss | peanuts (groundnuts) |
| Asparagus officinalis | Spargel | asparagus |
| Avena sativa | Hafer | oats |
| Beta vulgaris spp. altissima | Zuckerrübe | sugarbeets |
| Beta vulgaris spp. rapa | Futterrübe | fodder beets |
| Beta vulgaris spp. esculenta | Rote Rübe | table beets, red beets |
| Brassica napus var. napus | Raps | rape |
| Brassica napus var. napobrassica | Kohlrübe | |
| Brassica napus var rapa | Weisse Rübe | turnips |
| Brassica rapas var. silvestris | Rübsen | |
| Camellia sinensis | Teestrauch | tea plants |
| Carthamus tinctorius | Saflor – Färberdistel | safflower |
| Carya illinoinensis | Pekannussbaum | pecan trees |
| Citrus limon | Zitrone | lemon |
| Citrus maxima | Pampelmuse | grapefruits |
| Citrus reticulata | Mandarine | |
| Citrus sinensis | Apfelsine, Orange | orange trees |
| Coffea arabica (Coffea canephora, Coffea liberica) | Kaffee | coffe plants |
| Cucumis melo | Melone | melons |
| Cucumis sativus | Gurke | cucumber |
| Cynodon dactylon | Bermudagrass | Bermudagrass in turfs and lawn |
| Daucus carota | Möhre | carrots |
| Elaeis guineensis | Ölplame | oil palms |
| Fragaria vesca | Erdbeere | strawberries |
| Glycine max | Sojabohne | soybeans |
| Gossypium hirsutum (Gossypium arboreum, Gossypium herbaceum, Gossypium vitifolium) | Baumwolle | cotton |
| Helianthus annuus | Sonnenblume | sunflowers |
| Helianthus tuberosus | Topinambur | |

| Botanischer Name | Deutscher Name | Englischer Name |
|---|---|---|
| Hevea brasiliensis | Parakautschukbaum | rubber plants |
| Hordeum vulgare | Gerste | barley |
| Humulus lupulus | Hopfen | hop |
| Ipomoea batatas | Süsskartoffeln | sweet pottato |
| Juglans regia | Walnussbaum | walnut trees |
| Lactuca sativa | Kopfsalat | lettuce |
| Lens culinaris | Linse | lentils |
| Linum usitatissimum | Faserlein | flax |
| Lycopersicon lycopersicum | Tomate | tomato |
| Malus spp. | Apfel | apple trees |
| Manihot esculenta | Maniok | cassava |
| Medicago sativa | Luzerne | alfalfa (lucerne) |
| Mentha piperita | Pfefferminze | peppermint |
| Musa spp. | Obst- und Mehlbanane | banana plants |
| Nicotiana tabacum (N. rustica) | Tabak | tabacco |
| Olea europaea | Ölbaum | olive trees |
| Oryza sativa | Reis | rice |
| Panicum miliaceum | Rispenhirse | |
| Phaseolus lunatus | Mondbohne | limabeans |
| Phaseolus mungo | Urdbohne | mungbeans |
| Phaseolus vulgaris | Buschbohnen | snapbeans, green beans, dry beans |
| Pennisetum glaucum | Perl- oder Rohrkolbenhirse | |
| Petroselinum crispum spp. tuberosum | Wurzelpetersilie | parsley |
| Picea abies | Rotfichte | Norway spruce |
| Abies alba | Weisstanne | fir |
| Pinus spp. | Kiefer | pine trees |
| Pisum sativum | Gartenerbse | English peas |
| Prunus domestica | Pflaume | plum trees |
| Prunus dulcis | Mandelbaum | almond trees |
| Prunus persica | Pfirsich | peach trees |
| Pyrus communis | Birne | pear trees |
| Ribes sylvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sesamum indicum | Sesam | sesame |
| Solanum tuberosum | Kartoffel | Irish potatoes |
| Sorghum bicolor (s. vulgare) | Mohrenhirse | sorghum |
| Sorghum dochna | Zuckerhirse | |
| Spinacia oleracea | Spinat | spinach |
| Theobroma cacao | Kakaobaum | cacao plants |
| Trifolium pratense | Rotklee | red clover |
| Triticum aestivum | Weizen | wheat |
| Vaccinium corymbosum | Kulturheidelbeere | blueberry |
| Vicia faba | Pferdebohnen | tick beans |
| Vigna sinensis (V. unguiculata) | Kuhbohne | cow peas |
| Vitis vinifera | Weinrebe | grapes |
| Zea mays | Mais | Indian corn, sweet corn, maize |

Tabelle 1 – Liste der Pflanzennamen

| Botanischer Name | Abkürzung in Tabelle | Deutsche Bezeichnung | Englische Bezeichnung |
|---|---|---|---|
| Avena fatua | Avena fatua | Flughafer | wild oats |
| Centaurea cyanus | Centaurea cyan. | Kornblume | cornflower |
| Chenopodium spp. | Chenpod. spp. | Gänsefuss-Arten | lambsquarter |

Tabelle 1 (Fortsetzung)

| Botanischer Name | Abkürzung in Tabelle | Deutsche Bezeichnung | Englische Bezeichnung |
|---|---|---|---|
| Cyperus esculentus | | Erdmantel | yellow nutsedge |
| Cyperus iria | | | rice flatsedge |
| Datura stramonium | Datura stramon. | gemeiner Steckapfel | jimsonweed |
| Euphorbia geniculata | Euphorb. genic. | südamerikanische Wolfsmilchart | wild poinsetia |
| Matricaria chamomilla | Matric. cham. | echte Kamille | wild chamomile |
| Oryza sativa | Oryza sativa | Reis | rice |
| Sida spinosa | Sida spinosa | stachelige Samtmalve | teaweed |
| Solanum nigrum | Solan. nigr. | schwarzer Nachtschatten | blacknightshade |
| Zea mays | Zea mays | Mais | Indian corn |

Tabelle 2 – Selektive herbizide Wirkung bei Vorauflaufanwendung im Gewächshaus

| Wirkstoff | kg/ha | Testpflanzen und Schädigung % | | | | |
|---|---|---|---|---|---|---|
| | | Oryza sativa | Zea mays | Centaurea cyan. | Sida spinosa | Solanum nigrum |
| Nr. 1 | 1.0 | 0 | 15 | 98 | 95 | 95 |
| 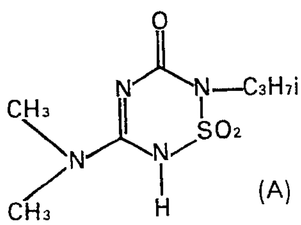 (A) bekannt | 1.0 | 55 | 32 | 50 | 50 | 90 |

0 = keine Schädigung
100 = Pflanzen nicht aufgelaufen oder abgestorben

Tabelle 3 – Selektive herbizide Wirkung bei Nachauflaufanwendung im Gewächshaus

| Wirkstoff | kg/ha | Testpflanzen und Schädigung % | | | | | | | |
|---|---|---|---|---|---|---|---|---|---|
| | | Oryza sativa | Zea mays | Avena fatua | Chenopod. spp. | Datura stramon. | Euphorb. genic. | Matric cham. | Solan nigr. |
| Nr. 1 | 1.0 | 8 | 10 | 82 | 83 | 100 | 98 | 80 | 100 |
| A bekannt | 1.0 | 32 | 25 | 62 | 55 | 80 | 20 | 60 | 77 |

0 = keine Schädigung
100 = Pflanzen nicht aufgelaufen oder abgestorben

**Patentansprüche** für die Vertragsstaaten: BE, CH, DE, FR, GB, IT, LI, LU, NL, SE

1. 6H-1,2,4,6-Thiatriazin-1,1-dioxid der Formel

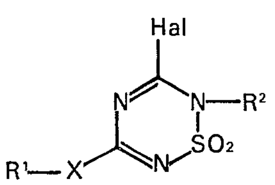

in der
$R^1$ einen gesättigten geradkettigen aliphatischen Rest mit 1 bis 10 Kohlenstoffatomen, einen cycloaliphatischen Rest mit 3 bis 7 Kohlenstoffatomen, einen verzweigten gesättigten oder ungesättigten aliphatischen Rest mit 3 bis 10 Kohlenstoffatomen, einen halogen-, alkoxy- oder alkylmercapto-substituierten geradkettigen oder verzweigten aliphatischen Rest mit 2 bis 10 Kohlenstoffatomen, einen cycloalkyloxy-substituierten aliphatischen Rest mit 4 bis 10 Kohlenstoffatomen, einen gegebenenfalls halogen-, niedrigalkyl-, niedrigalkoxy-substituierten Phenylrest oder einen gegebenenfalls halogensubstituierten Benzylrest, Allyl, Methallyl, Crotyl, 2-Ethyl-hexen-2-yl-1, Hexen-5-yl-1, 2-Methyl-buten-2-yl-1, 2-Methyl-buten-1-yl-3, Butin-1-yl-3, Butin-2-yl-1, Buten-1-yl-3, Propargyl, 2-Methyl-buten-1-yl-4, 2-Methyl-buten-2-yl-4, 3-Methyl-buten-1-yl-3, 1-Cyclohexylethyl,

$R^2$ Wasserstoff, einen geradkettigen aliphatischen Rest mit 1 bis 10 Kohlenstoffatomen, einen cycloaliphatischen Rest mit 3 bis 7 Kohlenstoffatomen, einen verzweigten aliphatischen Rest mit 3 bis 10 Kohlenstoffatomen oder einen halogen- oder alkoxy-substituierten Alkylrest mit 2 bis 10 Kohlenstoffatomen,

X Sauerstoff, Schwefel, eine Sulfinyl- oder Sulfonylgruppe und der Rest Hal ein Halogenatom bedeutet.

2. Herbizid, enthaltend ein 6H-1,2,4,6-Thiatriazin-1,1-dioxid der Formel

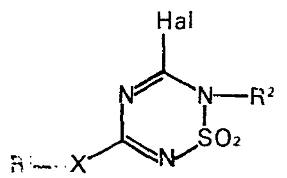

in der

$R^1$ einen gesättigten geradkettigen aliphatischen Rest mit 1 bis 10 Kohlenstoffatomen, einen cycloaliphatischen Rest mit 3 bis 7 Kohlenstoffatomen, einen verzweigten gesättigten oder ungesättigten aliphatischen Rest mit 3 bis 10 Kohlenstoffatomen, einen halogen-, alkoxy- oder alkylmercapto-substituierten geradkettigen oder verzweigten aliphatischen Rest mit 2 bis 10 Kohlenstoffatomen, einen cycloalkyloxy-substituierten aliphatischen Rest mit 4 bis 10 Kohlenstoffatomen, einen gegebenenfalls halogen-, niedrigalkyl-, niedrigalkoxy-substituierten Phenylrest oder einen gegebenenfalls halogensubstituierten Benzylrest, Allyl, Methallyl, Crotyl, 2-Ethyl-hexen-2-yl-1, Hexen-5-yl-1, 2-Methyl-buten-2-yl-1, 2-Methyl-buten-1-yl-3, Butin-1-yl-3, Butin-2-yl-1, Buten-1-yl-3, Propargyl, 2-Methyl-buten-1-yl-4, 2-Methyl-buten-2-yl-4, 3-Methyl-buten-1-yl-3, 1-Cyclohexylethyl,

$R^2$ Wasserstoff, einen geradkettigen aliphatischen Rest mit 1 bis 10 Kohlenstoffatomen, einen cycloaliphatischen Rest mit 3 bis 7 Kohlenstoffatomen, einen verzweigten aliphatischen Rest mit 3 bis 10 Kohlenstoffatomen oder einen halogen- oder alkoxy-substituierten Alkylrest mit 2 bis 10 Kohlenstoffatomen,

X Sauerstoff, Schwefel, eine Sulfinyl- oder Sulfonylgruppe und der Rest Hal ein Halogenatom bedeutet.

3. Herbizid, enthaltend einen festen oder flüssigen Trägerstoff und ein 6H-1,2,4,6-Thiatriazin-1,1-dioxid der Formel

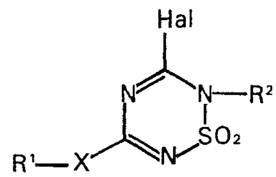

in der

$R^1$ einen gesättigten geradkettigen aliphatischen Rest mit 1 bis 10 Kohlenstoffatomen, einen cycloaliphatischen Rest mit 3 bis 7 Kohlenstoffatomen, einen verzweigten gesättigten oder ungesättigten aliphatischen Rest mit 3 bis 10 Kohlenstoffatomen, einen halogen-, alkoxy- oder alkylmercapto-substituierten geradkettigen oder verzweigten aliphatischen Rest mit 2 bis 10 Kohlenstoffatomen, einen cycloalkyloxy-substituierten aliphatischen Rest mit 4 bis 10 Kohlenstoffatomen, einen gegebenenfalls halogen-, niedrigalkyl-, niedrigalkoxy-substituierten Phenylrest oder einen gegebenenfalls halogensubstituierten Benzylrest, Allyl, Methallyl, Crotyl, 2-Ethyl-hexen-2-yl-1, Hexen-5-yl-1, 2-Methyl-buten-2-yl-1, 2-Methyl-buten-1-yl-3, Butin-1-yl-3, Butin-2-yl-1, Buten-1-yl-3, Propargyl, 2-Methyl-buten-1-yl-4, 2-Methyl-buten-2-yl-4, 3-Methyl-buten-1-yl-3, 1-Cyclohexylethyl,

$R^2$ Wasserstoff, einen geradkettigen aliphatischen Rest mit 1 bis 10 Kohlenstoffatomen, einen cycloaliphatischen Rest mit 3 bis 7 Kohlenstoffatomen, einen verzweigten aliphatischen Rest mit 3 bis 10 Kohlenstoffatomen oder einen halogen- oder alkoxy-substituierten Alkylrest mit 2 bis 10 Kohlenstoffatomen,

X Sauerstoff, Schwefel, eine Sulfinyl- oder Sulfonylgruppe und der Rest Hal ein Halogenatom bedeutet.

4. Verfahren zur Herstellung eines Herbizids, dadurch gekennzeichnet, dass man einen festen oder flüssigen Trägerstoff vermischt mit einem 6H-1,2,4,6-Thiatriazin-1,1-dioxid der Formel

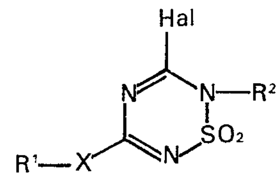

in der

$R^1$ einen gesättigten geradkettigen aliphatischen Rest mit 1 bis 10 Kohlenstoffatomen, einen cycloaliphatischen Rest mit 3 bis 7 Kohlenstoffatomen, einen verzweigten gesättigten oder ungesättigten aliphatischen Rest mit 3 bis 10 Kohlenstoffatomen, einen halogen-, alkoxy- oder alkylmercapto-substituierten geradkettigen oder verzweigten aliphatischen Rest mit 2 bis 10 Kohlenstoffatomen, einen cycloalkyloxy-substituierten aliphatischen Rest mit 4 bis 10 Kohlenstoffatomen, einen gegebenenfalls halogen-, niedrigalkyl-, niedrigalkoxy-substituierten Phenylrest oder einen gegebenenfalls halogensubstituierten Benzylrest, Allyl, Methallyl, Crotyl, 2-Ethyl-hexen-2-yl-1, Hexen-5-yl-1, 2-Methyl-buten-2-yl-1, 2-Methyl-buten-1-yl-3, Butin-1-yl-3, Butin-2-yl-1, Buten-1-yl-3, Propargyl, 2-Methyl-buten-1-yl-4, 2-Methyl-buten-2-yl-4, 3-Methyl-buten-1-yl-3, 1-Cyclohexylethyl,
$R^2$ Wasserstoff, einen geradkettigen aliphatischen Rest mit 1 bis 10 Kohlenstoffatomen, einen cylcoaliphatischen Rest mit 3 bis 7 Kohlenstoffatomen, einen verzweigten aliphatischen Rest mit 3 bis 10 Kohlenstoffatomen oder einen halogen- oder alkoxy-substituierten Alkylrest mit 2 bis 10 Kohlenstoffatomen,
X Sauerstoff, Schwefel, eine Sulfinyl- oder Sulfonylgruppe und der Rest Hal ein Halogenatom bedeutet.

5. Verfahren zur Bekämpfung unerwünschten Pflanzenwuchses, dadurch gekennzeichnet, dass man die Pflanzen oder den Boden behandelt mit einem 6H-1,2,4,6-Thiatriazin-1,1-dioxid der Formel

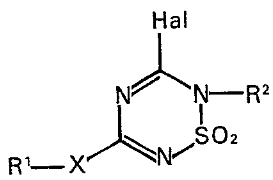

in der
$R^1$ einen gesättigten geradkettigen aliphatischen Rest mit 1 bis 10 Kohlenstoffatomen, einen cycloaliphatischen Rest mit 3 bis 7 Kohlenstoffatomen, einen verzweigten gesättigten oder ungesättigten aliphatischen Rest mit 3 bis 10 Kohlenstoffatomen, einen halogen-, alkoxy-oder alkylmercapto-substituierten geradkettigen oder verzweigten aliphatischen Rest mit 2 bis 10 Kohlenstoffatomen, einen cycloalkyloxy-substituierten aliphatischen Rest mit 4 bis 10 Kohlenstoffatomen, einen gegebenenfalls halogen-, niedrigalkyl-, niedrigalkoxy-substituierten Phenylrest oder einen gegebenenfalls halogensubstituierten Benzylrest, Allyl, Methallyl, Crotyl, 2-Ethyl-hexen-2-yl-1, Hexen-5-yl-1, 2-Methyl-buten-2-yl-1, 2-Methyl-buten-1-yl-3, Butin-1-yl-3, Butin-2-yl-1, Buten-1-yl-3, Propargyl, 2-Methyl-buten-1-yl-4, 2-Methyl-buten-2-yl-4, 3-Methyl-buten-1-yl-3, 1-Cyclohexylethyl,
$R^2$ Wasserstoff, einen geradkettigen aliphatischen Rest mit 1 bis 10 Kohlenstoffatomen, einen cylcoaliphatischen Rest mit 3 bis 7 Kohlenstoffatomen, einen verzweigten aliphatischen Rest mit 3 bis 10 Kohlenstoffatomen oder einen halogen- oder alkoxy-substituierten Alkylrest mit 2 bis 10 Kohlenstoffatomen,
X Sauerstoff, Schwefel, eine Sulfinyl- oder Sulfonylgruppe und der Rest Hal ein Halogenatom bedeutet.

6. Verfahren zur Herstellung eines 6H-1,2,4,6-Thiatriazin-1,1-dioxid der Formel

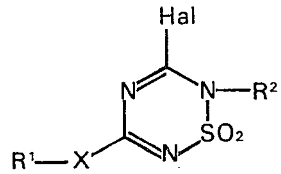

in der
$R^1$ einen gesättigten geradkettigen aliphatischen Rest mit 1 bis 10 Kohlenstoffatomen, einen cycloaliphatischen Rest mit 3 bis 7 Kohlenstoffatomen, einen verzweigten gesättigten oder ungesättigten aliphatischen Rest mit 3 bis 10 Kohlenstoffatomen, einen halogen-, alkoxy- oder alkylmercapto-substituierten geradkettigen oder verzweigten aliphatischen Rest mit 2 bis 10 Kohlenstoffatomen, einen cycloalkyloxy-substituierten aliphatischen Rest mit 4 bis 10 Kohlenstoffatomen, einen gegebenenfalls halogen-, niedrigalkyl-, niedrigalkoxy-substituierten Phenylrest oder einen gegebenenfalls halogensubstituierten Benzylrest, Allyl, Methallyl, Crotyl, 2-Ethyl-hexen-2-yl-1, Hexen-5-yl-1, 2-Methyl-buten-2-yl-1, 2-Methyl-buten-1-yl-3, Butin-1-yl-3, Butin-2-yl-1, Buten-1-yl-3, Propargyl, 2-Methyl-buten-1-yl-4, 2-Methyl-buten-2-yl-4, 3-Methyl-buten-1-yl-3, 1-Cyclohexylethyl,
$R^2$ Wasserstoff, einen geradkettigen aliphatischen Rest mit 1 bis 10 Kohlenstoffatomen, einen cycloaliphatischen Rest mit 3 bis 7 Kohlenstoffatomen, einen verzweigten aliphatischen Rest mit 3 bis 10 Kohlenstoffatomen oder einen halogen- oder alkoxy-substituierten Alkylrest mit 2 bis 10 Kohlenstoffatomen,
X Sauerstoff, Schwefel, eine Sulfinyl- oder Sulfonylgruppe und der Rest Hal ein Halogenatom bedeutet, dadurch gekennzeichnet, dass man eine Verbindung der Formel

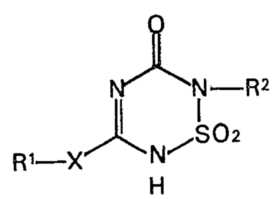

worin $R^1$, $R^2$ und X die oben genannte Bedeutung haben, oder deren Alkalisalze oder Erdalkalisalze mit einem Säurehalogenid der Phosphorsäure, phosphorigen Säure, Kohlensäure, Oxalsäure oder schwefligen Säure, gegebenenfalls in Gegenwart eines Lösungs- oder Verdünnungsmittels und gegebenenfalls in Gegenwart eines Reaktionsbeschleunigers umsetzt.

**Patentansprüche** für den Vertragsstaat: AT

1. Herbizid, enthaltend ein 6H-1,2,4,6-Thiatriazin-1,1-dioxid der Formel

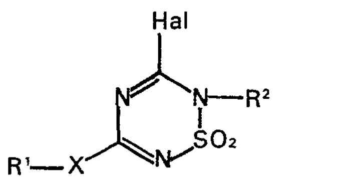

in der
$R^1$ einen gesättigten geradkettigen aliphatischen Rest mit 1 bis 10 Kohlenstoffatomen, einen cycloaliphatischen Rest mit 3 bis 7 Kohlenstoffatomen, einen verzweigten gesättigten oder ungesättigten aliphatischen Rest mit 3 bis 10 Kohlenstoffatomen, einen halogen-, alkoxy- oder alkylmercapto-substituierten geradkettigen oder verzweigten aliphatischen Rest mit 2 bis 10 Kohlenstoffatomen, einen cycloalkyloxy-substituierten aliphatischen Rest mit 4 bis 10 Kohlenstoffatomen, einen gegebenenfalls halogen-, niedrigalkyl-, niedrigalkoxy-substituierten Phenylrest oder einen gegebenenfalls halogensubstituierten Benzylrest, Allyl, Methallyl, Crotyl, 2-Ethyl-hexen-2-yl-1, Hexen-5-yl-1, 2-Methyl-buten-2-yl-1, 2-Methyl-buten-1-yl-3, Butin-1-yl-3, Butin-2-yl-1, Buten-1-yl-3, Propargyl, 2-Methyl-buten-1-yl-4, 2-Methyl-buten-2-yl-4, 3-Methyl-buten-1-yl-3, 1-Cyclohexylethyl,
$R^2$ Wasserstoff, einen geradkettigen aliphatischen Rest mit 1 bis 10 Kohlenstoffatomen, einen cycloaliphatischen Rest mit 3 bis 7 Kohlenstoffatomen, einen verzweigten aliphatischen Rest mit 3 bis 10 Kohlenstoffatomen oder einen halogen- oder alkoxy-substituierten Alkylrest mit 2 bis 10 Kohlenstoffatomen,
X Sauerstoff, Schwefel, eine Sulfinyl- oder Sulfonylgruppe und der Rest Hal ein Halogenatom bedeutet.

2. Herbizid, enthaltend einen festen oder flüssigen Trägerstoff und ein 6H-1,2,4,6-Thiatriazin-1,1-dioxid der Formel

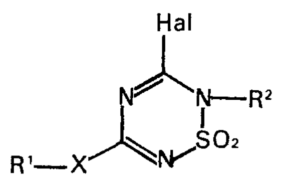

in der
$R^1$ einen gesättigten geradkettigen aliphatischen Rest mit 1 bis 10 Kohlenstoffatomen, einen cycloaliphatischen Rest mit 3 bis 7 Kohlenstoffatomen, einen verzweigten gesättigten oder ungesättigten aliphatischen Rest mit 3 bis 10 Kohlenstoffatomen, einen halogen-, alkoxy- oder alkylmercapto-substituierten geradkettigen oder verzweigten aliphatischen Rest mit 2 bis 10 Kohlenstoffatomen, einen cycloalkyloxy-substituierten aliphatischen Rest mit 4 bis 10 Kohlenstoffatomen, einen gegebenenfalls halogen-, niedrigalkyl-, niedrigalkoxy-substituierten Phenylrest oder einen gegebenenfalls halogensubstituierten Benzylrest, Allyl, Methallyl, Crotyl, 2-Ethyl-hexen-2-yl-1, Hexen-5-yl-1, 2-Methyl-buten-2-yl-1, 2-Methyl-buten-1-yl-3, Butin-1-yl-3, Butin-2-yl-1, Buten-1-yl-3, Propargyl, 2-Methyl-buten-1-yl-4, 2-Methyl-buten-2-yl-4, 3-Methyl-buten-1-yl-3, 1-Cyclohexylethyl,
$R^2$ Wasserstoff, einen geradkettigen aliphatischen Rest mit 1 bis 10 Kohlenstoffatomen, einen cycloaliphatischen Rest mit 3 bis 7 Kohlenstoffatomen, einen verzweigten aliphatischen Rest mit 3 bis 10 Kohlenstoffatomen oder einen halogen- oder alkoxy-substituierten Alkylrest mit 2 bis 10 Kohlenstoffatomen,
X Sauerstoff, Schwefel, eine Sulfinyl- oder Sulfonylgruppe und der Rest Hal ein Halogenatom bedeutet.

3. Verfahren zur Herstellung eines Herbizids, dadurch gekennzeichnet, dass man einen festen oder flüssigen Trägerstoff vermischt mit einem 6H-1,2,4,6-Thiatriazin-1,1-dioxid der Formel

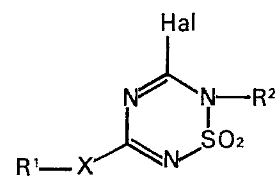

in der
$R^1$ einen gesättigten geradkettigen aliphatischen Rest mit 1 bis 10 Kohlenstoffatomen, einen cycloaliphatischen Rest mit 3 bis 7 Kohlenstoffatomen, einen verzweigten gesättigten oder ungesättigten aliphatischen Rest mit 3 bis 10 Kohlenstoffatomen, einen halogen-, alkoxy- oder alkylmercapto-substituierten geradkettigen oder verzweigten aliphatischen Rest mit 2 bis 10 Kohlenstoffatomen, einen cycloalkyloxy-substituierten aliphatischen Rest mit 4 bis 10 Kohlenstoffatomen, einen gegebenenfalls halogen-, niedrigalkyl-, niedrigalkoxy-substituierten Phenylrest oder einen gegebenenfalls halogensubstituierten Benzylrest, Allyl, Methallyl, Crotyl, 2-Ethyl-hexen-2-yl-1, Hexen-5-yl-1, 2-Methyl-buten-2-yl-1, 2-Methyl-buten-1-yl-3, Butin-1-yl-3, Butin-2-yl-1, Buten-1-yl-3, Propargyl, 2-Methyl-buten-1-yl-4, 2-Methyl-buten-2-yl-4, 3-Methyl-buten-1-yl-3, 1-Cyclohexylethyl,
$R^2$ Wasserstoff, einen geradkettigen aliphatischen Rest mit 1 bis 10 Kohlenstoffatomen, einen cycloaliphatischen Rest mit 3 bis 7 Kohlenstoffatomen, einen verzweigten aliphatischen Rest mit 3 bis 10 Kohlenstoffatomen oder einen halogen- oder alkoxy-substituierten Alkylrest mit 2 bis 10 Kohlenstoffatomen,
X Sauerstoff, Schwefel, eine Sulfinyl- oder Sulfonylgruppe und der Rest Hal ein Halogenatom bedeutet.

4. Verfahren zur Bekämpfung unerwünschten Pflanzenwuchses, dadurch gekennzeichnet, dass man die Pflanzen oder den Boden behandelt mit einem 6H-1,2,4,6-Thiatriazin-1,1-dioxid der Formel

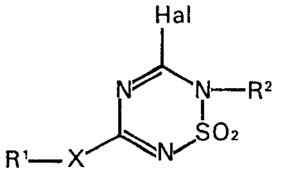

in der
$R^1$ einen gesättigten geradkettigen aliphatischen Rest mit 1 bis 10 Kohlenstoffatomen, einen cycloaliphatischen Rest mit 3 bis 7 Kohlenstoffatomen, einen verzweigten gesättigten oder ungesättigten aliphatischen Rest mit 3 bis 10 Kohlenstoffatomen, einen halogen-, alkoxy- oder alkylmercapto-substituierten geradkettigen oder verzweigten aliphatischen Rest mit 2 bis 10 Kohlenstoffatomen, einen cycloalkyloxy-substituierten aliphatischen Rest mit 4 bis 10 Kohlenstoffatomen, einen gegebenenfalls halogen-, niedrigalkyl-, niedrigalkoxy-substituierten Phenylrest oder einen gegebenenfalls halogensubstituierten Benzylrest, Allyl, Methallyl, Crotyl, 2-Ethyl-hexen-2-yl-1, Hexen-5-yl-1, 2-Methyl-buten-2-yl-1, 2-Methyl-buten-1-yl-3, Butin-1-yl-3, Butin-2-yl-1, Buten-1-yl-3, Propargyl, 2-Methyl-buten-1-yl-4, 2-Methyl-buten-2-yl-4, 3-Methyl-buten-1-yl-3, 1-Cyclohexylethyl,
$R^2$ Wasserstoff, einen geradkettigen aliphatischen Rest mit 1 bis 10 Kohlenstoffatomen, einen cycloaliphatischen Rest mit 3 bis 7 Kohlenstoffatomen, einen verzweigten aliphatischen Rest mit 3 bis 10 Kohlenstoffatomen oder einen halogen- oder alkoxy-substituierten Alkylrest mit 2 bis 10 Kohlenstoffatomen,
X Sauerstoff, Schwefel, eine Sulfinyl- oder Sulfonylgruppe und der Rest Hal ein Halogenatom bedeutet.

5. Verfahren zur Herstellung eines 6H-1,2,4,6-Thiatriazin-1,1-dioxid der Formel

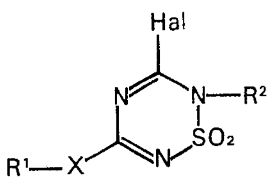

in der
$R^1$ einen gesättigten geradkettigen aliphatischen Rest mit 1 bis 10 Kohlenstoffatomen, einen cycloaliphatischen Rest mit 3 bis 7 Kohlenstoffatomen, einen verzweigten gesättigten oder ungesättigten aliphatischen Rest mit 3 bis 10 Kohlenstoffatomen, einen halogen-, alkoxy- oder alkylmercapto-substituierten geradkettigen oder verzweigten aliphatischen Rest mit 2 bis 10 Kohlenstoffatomen, einen cycloalkyloxy-substituierten aliphatischen Rest mit 4 bis 10 Kohlenstoffatomen, einen gegebenenfalls halogen-, niedrigalkyl-, niedrigalkoxy-substituierten Phenylrest oder einen gegebenenfalls halogensubstituierten Benzylrest, Allyl, Methallyl, Crotyl, 2-Ethyl-hexen-2-yl-1, Hexen-5-yl-1, 2-Methyl-buten-2-yl-1, 2-Methyl-buten-1-yl-3, Butin-1-yl-3, Butin-2-yl-1, Buten-1-yl-3, Propargyl, 2-Methyl-buten-1-yl-4, 2-Methyl-buten-2-yl-4, 3-Methyl-buten-1-yl-3, 1-Cyclohexylethyl,
$R^2$ Wasserstoff, einen geradkettigen aliphatischen Rest mit 1 bis 10 Kohlenstoffatomen, einen cycloaliphatischen Rest mit 3 bis 7 Kohlenstoffatomen, einen verzweigten aliphatischen Rest mit 3 bis 10 Kohlenstoffatomen oder einen halogen- oder alkoxy-substituierten Alkylrest mit 2 bis 10 Kohlenstoffatomen,
X Sauerstoff, Schwefel, eine Sulfinyl- oder Sulfonylgruppe und der Rest Hal ein Halogenatom bedeutet, dadurch gekennzeichnet, dass man eine Verbindung der Formel

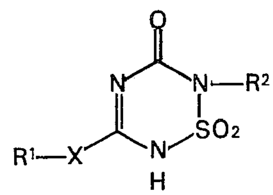

worin $R^1$, $R^2$ und X die oben genannte Bedeutung haben, oder deren Alkalisalze oder Erdalkalisalze mit einem Säurehalogenid der Phosphorsäure, phosphorigen Säure, Kohlensäure, Oxalsäure oder schwefligen Säure, gegebenenfalls in Gegenwart eines Lösungs- oder Verdünnungsmittels und gegebenenfalls in Gegenwart eines Reaktionsbeschleunigers umsetzt.

**Claims** for the Contracting States BE to SE

1. A 6H-1,2,4,6-thiatriazine-1,1-dioxide of the formula

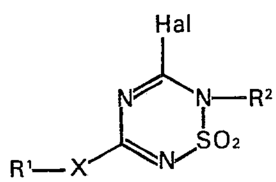
,

where
$R^1$ is a saturated straight-chain aliphatic radical of 1 to 10 carbon atoms, a cycloaliphatic radical of 3 to 7 carbon atoms, a branched, saturated or unsaturated aliphatic radical of 3 to 10 carbon atoms, a halogen-, alkoxy- or alkylmercapto-substituted straight-chain or branched aliphatic radical of 2 to 10 carbon atoms, a cycloalkyloxy-substituted aliphatic radical of 4 to 10 carbon atoms, unsubstituted or halogen-, lower alkyl- or lower alkoxy-substituted phenyl, or unsubstituted or halogen-substituted benzyl, allyl, methallyl, crotyl, 2-ethyl-hex-2-en-1-yl, hex-5-en-1-yl, 2-methyl-but-2-en-1-yl, 2-methyl-but-1-en-3-yl, but-1-yn-3-yl, but-2-yn-1-yl, but-1-en-3-yl, propargyl, 2-methyl-but-1-en-4-yl, 2-methyl-but-2-en-4-yl, 3-methyl-but-1-en-3-yl and 1-cyclohexylethyl,
$R^2$ is hydrogen, a straight-chain aliphatic radical of 1 to 10 carbon atoms, a cycloaliphatic radical of 3 to 7 carbon atoms, a branched aliphatic radical of 3 to 10 carbon atoms or halogen- or alkoxy-substituted alkyl of 2 to 10 carbon atoms,
X is oxygen, sulfur, sulfinyl or sulfonyl, and Hal is halogen.

2. A herbicide containing a 6H-1,2,4,6-thiatriazine-1,1-dioxide of the formula

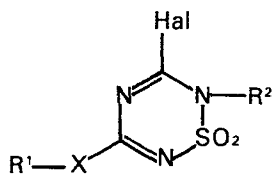
,

where
$R^1$ is a saturated straight-chain aliphatic radical of 1 to 10 carbon atoms, a cycloaliphatic radical of 3 to 7 carbon atoms, a branched, saturated or unsaturated aliphatic radical of 3 to 10 carbon atoms, a halogen-, alkoxy- or alkylmercapto-substituted straight-chain or branched aliphatic radical of 2 to 10 carbon atoms, a cycloalkyloxy-substituted aliphatic radical of 4 to 10 carbon atoms, unsubstituted or halogen-, lower alkyl- or lower alkoxy-substituted phenyl, or unsubstituted or halogen-substituted benzyl, allyl, methallyl, crotyl, 2-ethyl-hex-2-en-1-yl, hex-5-en-1-yl, 2-methyl-but-2-en-1-yl, 2-methyl-but-1-en-3-yl, but-1-yn-3-yl, but-2-yn-1-yl, but-1-en-3-yl, propargyl, 2-methyl-but-1-en-4-yl, 2-methyl-but-2-en-4-yl, 3-methyl-but-1-en-3-yl and 1-cyclohexylethyl,
$R^2$ is hydrogen, a straight-chain aliphatic radical of 1 to 10 carbon atoms, a cycloaliphatic radical of 3 to 7 carbon atoms, a branched aliphatic radical of 3 to 10 carbon atoms or halogen- or alkoxy-substituted alkyl of 2 to 10 carbon atoms,
X is oxygen, sulfur, sulfinyl or sulfonyl, and Hal is halogen.

3. A herbicide containing a solid or liquid carrier and a 6H-1,2,4,6-thiatriazine-1,1-dioxide of the formula

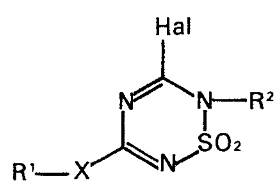
,

where
$R^1$ is a saturated straight-chain aliphatic radical of 1 to 10 carbon atoms, a cycloaliphatic radical of 3 to 7 carbon atoms, a branched, saturated or unsaturated aliphatic radical of 3 to 10 carbon atoms, a halogen-, alkoxy- or alkylmercapto-substituted straight-chain or branched aliphatic radical of 2 to 10 carbon atoms, a cycloalkyloxy-substituted aliphatic radical of 4 to 10 carbon atoms, unsubstituted or halogen-, lower alkyl- or lower alkoxy-substituted phenyl, or unsubstituted or halogen-substituted benzyl, allyl, methallyl, crotyl, 2-ethyl-hex-2-en-1-yl, hex-5-en-1-yl, 2-methyl-but-2-en-1-yl, 2-methyl-but-1-en-3-yl, but-1-yn-3-yl, but-2-yn-1-yl, but-1-en-3-yl, propargyl, 2-methyl-but-1-en-4-yl, 2-methyl-but-2-en-4-yl, 3-methyl-but-1-en-3-yl and 1-cyclohxylethyl,
$R^2$ is hydrogen, a straight-chain aliphatic radical of 1 to 10 carbon atoms, a cycloaliphatic radical of 3 to 7 carbon atoms, a branched aliphatic radical of 3 to 10 carbon atoms or halogen- or alkoxy-substituted alkyl of 2 to 10 carbon atoms,
X is oxygen, sulfur, sulfinyl or sulfonyl, and Hal is halogen.

4. A process for the production of a herbicide, wherein a solid or liquid carrier is mixed with a 6H-1,2,4,6-thiatriazine-1,1-dioxide of the formula

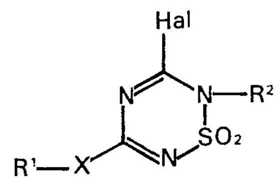
,

where
$R^1$ is a saturated straight-chain aliphatic radical of 1 to 10 carbon atoms, a cycloaliphatic radical of 3 to 7 carbon atoms, a branched, saturated or unsaturated aliphatic radical of 3 to 10 carbon atoms, a halogen-, alkoxy- or alkylmercapto-substituted straight-chain or branched aliphatic radical of 2 to 10 carbon atoms, a cycloalkyloxy-substituted aliphatic radical of 4 to 10 carbon atoms, unsubstituted or halogen-, lower alkyl- or lower alkoxy-substituted phenyl, or unsubstituted or halogen-substituted benzyl, allyl, methallyl, crotyl, 2-ethyl-hex-2-en-1-yl, hex-5-en-1-yl, 2-methyl-but-2-en-1-yl, 2-methyl-but-1-en-3-yl, but-1-yn-3-yl, but-2-yn-1-yl, but-1-en-3-yl, propargyl, 2-methyl-but-1-en-4-yl, 2-methyl-but-2-en-4-yl, 3-methyl-but-1-en-3-yl and 1-cyclohexylethyl,
$R^2$ is hydrogen, a straight-chain aliphatic radical of 1 to 10 carbon atoms, a cycloaliphatic radical of 3 to 7 carbon atoms, a branched aliphatic radical of 3 to 10 carbon atoms or halogen- or alkoxy-substituted alkyl of 2 to 10 carbon atoms,
X is oxygen, sulfur, sulfinyl or sulfonyl, and Hal is halogen.

5. A process for controlling unwanted plant growth, wherein the plants or the soil are treated with a 6H-1,2,4,6-thiatriazine-1,1-dioxide of the formula

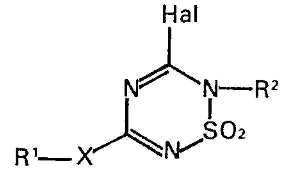
,

where
$R^1$ is a saturated straight-chain aliphatic radical of 1 to 10 carbon atoms, a cycloaliphatic radical of 3 to 7 carbon atoms, a branched, saturated or unsaturated aliphatic radical of 3 to 10 carbon atoms, a halogen-, alkoxy- or alkylmercapto-substituted straight-chain or branched aliphatic radical of 2 to 10 carbon atoms, a cycloalkyloxy-substituted aliphatic radical of 4 to 10 carbon atoms,

unsubstituted or halogen-, lower alkyl- or lower alkoxy-substituted phenyl, or unsubstituted or halogen-substituted benzyl, allyl, methallyl, crotyl, 2-ethyl-hex-2-en-1-yl, hex-5-en-1-yl, 2-methyl-but-2-en-1-yl, 2-methyl-but-1-en-3-yl, but-1-yn-3-yl, but-2-yn-1-yl, but-1-en-3-yl, propargyl, 2-methyl-but-1-en-4-yl, 2-methyl-but-2-en-4-yl, 3-methyl-but-1-en-3-yl and 1-cyclohexylethyl,
$R^2$ is hydrogen, a straight-chain aliphatic radical of 1 to 10 carbon atoms, a cycloaliphatic radical of 3 to 7 carbon atoms, a branched aliphatic radical of 3 to 10 carbon atoms or halogen- or alkoxy-substituted alkyl of 2 to 10 carbon atoms,
X is oxygen, sulfur, sulfinyl or sulfonyl, and Hal is halogen.

6. A process for the preparation of a 6H-1,2,4,6-thiatriazine-1,1-dioxide of the formula

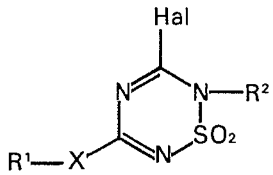,

where
$R^1$ is a saturated straight-chain aliphatic radical of 1 to 10 carbon atoms, a cycloaliphatic radical of 3 to 7 carbon atoms, a branched, saturated or unsaturated aliphatic radical of 3 to 10 carbon atoms, a halogen-, alkoxy- or alkylmercapto-substituted straight-chain or branched aliphatic radical of 2 to 10 carbon atoms, a cycloalkyloxy-substituted aliphatic radical of 4 to 10 carbon atoms, unsubstituted or halogen-, lower alkyl- or lower alkoxy-substituted phenyl, or unsubstituted or halogen-substituted benzyl, allyl, methallyl, crotyl, 2-ethyl-hex-2-en-1-yl, hex-5-en-1-yl, 2-methyl-but-2-en-1-yl, 2-methyl-but-1-en-3-yl, but-1-yn-3-yl, but-2-yn-1-yl, but-1-en-3-yl, propargyl, 2-methyl-but-1-en-4-yl, 2-methyl-but-2-en-4-yl, 3-methyl-but-1-en-3-yl and 1-cyclohexylethyl,
$R^2$ is hydrogen, a straight-chain aliphatic radical of 1 to 10 carbon atoms, a cycloaliphatic radical of 3 to 7 carbon atoms, a branched aliphatic radical of 3 to 10 carbon atoms or halogen- or alkoxy-substituted alkyl of 2 to 10 carbon atoms,
X is oxygen, sulfur, sulfinyl or sulfonyl, and Hal is halogen,
wherein a compound of the formula

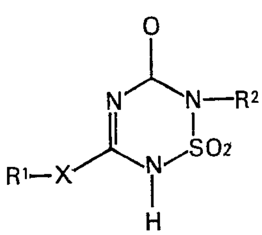,

where $R^1$, $R^2$ and X have the above meanings, or an alkali metal salt or alkaline earth metal salt thereof, is reacted with an acid halide of phosphoric acid, phosphorus acid, carbonic acid, oxalic acid or sulfurous acid, in the presence or absence of a solvent or diluent and in the presence or absence of a reaction accelerator.

**Claims** for the Contracting State AT

1. A herbicide containing a 6H-1,2,4,6-thiatriazine-1,1-dioxide of the formula

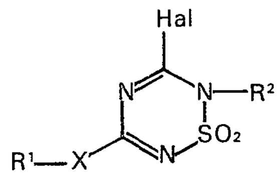,

where
$R^1$ is a saturated straight-chain aliphatic radical of 1 to 10 carbon atoms, a cycloaliphatic radical of 3 to 7 carbon atoms, a branched, saturated or unsaturated aliphatic radical of 3 to 10 carbon atoms, a halogen-, alkoxy- or alkylmercapto-substituted straight-chain or branched aliphatic radical of 2 to 10 carbon atoms, a cycloalkyloxy-substituted aliphatic radical of 4 to 10 carbon atoms, unsubstituted or halogen-, lower alkyl- or lower alkoxy-substituted phenyl, or unsubstituted or halogen-substituted benzyl, allyl, methallyl, crotyl, 2-ethyl-hex-2-en-1-yl, hex-5-en-1-yl, 2-methyl-but-2-en-1-yl, 2-methyl-but-1-en-3-yl, but-1-yn-3-yl, but-2-yn-1-yl, but-1-en-3-yl, propargyl, 2-methyl-but-1-en-4-yl, 2-methyl-but-2-en-4-yl, 3-methyl-but-1-en-3-yl and 1-cyclohexylethyl,
$R^2$ is hydrogen, a straight-chain aliphatic radical of 1 to 10 carbon atoms, a cycloaliphatic radical of 3 to 7 carbon atoms, a branched aliphatic radical of 3 to 10 carbon atoms or halogen- or alkoxy-substituted alkyl of 2 to 10 carbon atoms,
X is oxygen, sulfur, sulfinyl or sulfonyl, and Hal is halogen.

2. A herbicide containing a solid or liquid carrier and a 6H-1,2,4,6-thiatriazine-1,1-dioxide of the formula

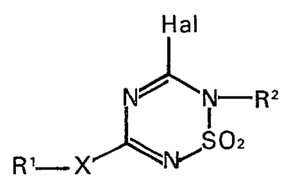,

where
$R^1$ is a saturated straight-chain aliphatic radical of 1 to 10 carbon atoms, a cycloaliphatic radical of 3 to 7 carbon atoms, a branched, saturated or unsaturated aliphatic radical of 3 to 10 carbon atoms, a halogen-, alkoxy- or alkylmercapto-substituted straight-chain or branched aliphatic radical of 2 to 10 carbon atoms, a cycloalkyloxy-substituted aliphatic radical of 4 to 10 carbon atoms, unsubstituted or halogen-, lower alkyl- or lower alkoxy-substituted phenyl, or unsubstituted or halogen-substituted benzyl, allyl, methallyl, crotyl, 2-ethyl-hex-2-en-1-yl, hex-5-en-1-yl, 2-methyl-but-2-en-1-yl, 2-methyl-but-1-en-3-yl, but-1-yn-3-yl, but-2-yn-1-yl, but-1-en-3-yl, propargyl, 2-methyl-but-1-en-4-yl, 2-methyl-but-2-en-4-yl, 3-methyl-but-1-en-3-yl and 1-cyclohexylethyl,
$R^2$ is hydrogen, a straight-chain aliphatic radical of 1 to 10 carbon atoms, a cycloaliphatic radical of 3 to 7 carbon atoms, a branched aliphatic radical of 3 to 10 carbon atoms or halogen- or alkoxy-substituted alkyl of 2 to 10 carbon atoms,
X is oxygen, sulfur, sulfinyl or sulfonyl, and Hal is halogen.

3. A process for the production of a herbicide, wherein a solid or liquid carrier is mixed with a 6H-1,2,4,6-thiatriazine-1,1-dioxide of the formula

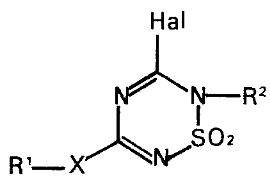
,

where
$R^1$ is a saturated straight-chain aliphatic radical of 1 to 10 carbon atoms, a cycloaliphatic radical of 3 to 7 carbon atoms, a branched, saturated or unsaturated aliphatic radical of 3 to 10 carbon atoms, a halogen-, alkoxy- or alkylmercapto-substituted straight-chain or branched aliphatic radical of 2 to 10 carbon atoms, a cycloalkyloxy-substituted aliphatic radical of 4 to 10 carbon atoms, unsubstituted or halogen-, lower alkyl- or lower alkoxy-substituted phenyl, or unsubstituted or halogen-substituted benzyl, allyl, methallyl, crotyl, 2-ethyl-hex-2-en-1-yl, hex-5-en-1-yl, 2-methyl-but-2-en-1-yl, 2-methyl-but-1-en-3-yl, but-1-yn-3-yl, but-2-yn-1-yl, but-1-en-3-yl, propargyl, 2-methyl-but-1-en-4-yl, 2-methyl-but-2-en-4-yl, 3-methyl-but-1-en-3-yl and 1-cyclohexylethyl,
$R^2$ is hydrogen, a straight-chain aliphatic radical of 1 to 10 carbon atoms, a cycloaliphatic radical of 3 to 7 carbon atoms, a branched aliphatic radical of 3 to 10 carbon atoms or halogen- or alkoxy-substituted alkyl of 2 to 10 carbon atoms,
X is oxygen, sulfur, sulfinyl or sulfonyl, and Hal is halogen.

4. A process for controlling unwanted plant growth, wherein the plants or the soil are treated with a 6H-1,2,4,6-thiatriazine-1,1-dioxide of the formula

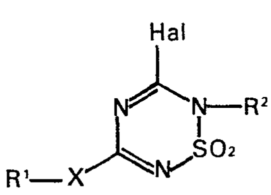
,

where
$R^1$ is a saturated straight-chain aliphatic radical of 1 to 10 carbon atoms, a cycloaliphatic radical of 3 to 7 carbon atoms, a branched, saturated or unsaturated aliphatic radical of 3 to 10 carbon atoms, a halogen-, alkoxy- or alkylmercapto-substituted straight-chain or branched aliphatic radical of 2 to 10 carbon atoms, a cycloalkyloxy-substituted aliphatic radical of 4 to 10 carbon atoms, unsubstituted or halogen-, lower alkyl- or lower alkoxy-substituted phenyl, or unsubstituted or halogen-substituted benzyl, allyl, methallyl, crotyl, 2-ethyl-hex-2-en-1-yl, hex-5-en-1-yl, 2-methyl-but-2-en-1-yl, 2-methyl-but-1-en-3-yl, but-1-yn-3-yl, but-2-yn-1-yl, but-1-en-3-yl, propargyl, 2-methyl-but-1-en-4-yl, 2-methyl-but-2-en-4-yl, 3-methyl-but-1-en-3-yl and 1-cyclohexylethyl,
$R^2$ is hydrogen, a straight-chain aliphatic radical of 1 to 10 carbon atoms, a cycloaliphatic radical of 3 to 7 carbon atoms, a branched aliphatic radical of 3 to 10 carbon atoms or halogen- or alkoxy-substituted alkyl of 2 to 10 carbon atoms,
X is oxygen, sulfur, sulfinyl or sulfonyl, and Hal is halogen.

5. A process for preparation of a 6H-1,2,4,6-thiatriazine-1,1-dioxide of the formula

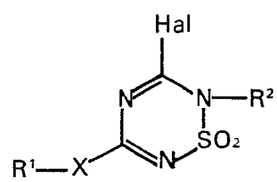
,

where
$R^1$ is a saturated straight-chain aliphatic radical of 1 to 10 carbon atoms, a cycloaliphatic radical of 3 to 7 carbon atoms, a branched, saturated or unsaturated aliphatic radical of 3 to 10 carbon atoms, a halogen-, alkoxy- or alkylmercapto-substituted straight-chain or branched aliphatic radical of 2 to 10 carbon atoms, a cycloalkyloxy-substituted aliphatic radical of 4 to 10 carbon atoms, unsubstituted or halogen-, lower alkyl- or lower alkoxy-substituted phenyl, or unsubstituted or halogen-substituted benzyl, allyl, methallyl, crotyl, 2-ethyl-hex-2-en-1-yl, hex-5-en-1-yl, 2-methyl-but-2-en-1-yl, 2-methyl-but-1-en-3-yl, but-1-yn-3-yl, but-2-yn-1-yl, but-1-en-3-yl, propargyl, 2-methyl-but-1-en-4-yl, 2-methyl-but-2-en-4-yl, 3-methyl-but-1-en-3-yl and 1-cyclohexylethyl,
$R^2$ is hydrogen, a straight-chain aliphatic radical of 1 to 10 carbon atoms, a cycloaliphatic radical of 3 to 7 carbon atoms, a branched aliphatic radical of 3 to 10 carbon atoms or halogen- or alkoxy-substituted alkyl of 2 to 10 carbon atoms,
X is oxygen, sulfur, sulfinyl or sulfonyl, and Hal is halogen,
wherein a compound of the formula

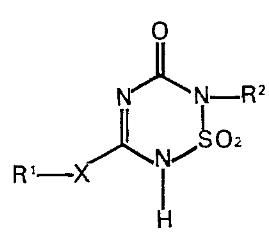

where $R^1$, $R^2$ and X have the above meanings, or an alkali metal salt or alkaline earth metal salt thereof, is reacted with an acid halide of phosphoric acid, phosphorus acid, carbonic acid, oxalic acid or sulfurous acid, in the presence or absence of a solvent or diluent and in the presence or absence of a reaction accelerator.

**Revendications** pour les Etats contractants BE à SE

1. 6H-1,2,4,6-thiatriazin-1,1-dioxyde de formule

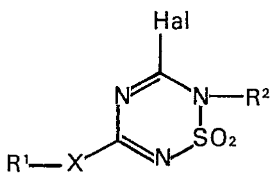

dans laquelle
$R^1$ représente un reste aliphatique saturé, à chaîne droite, ayant 1 à 10 atomes de carbone, un reste cycloaliphatique à 3 à 7 atomes de carbone, un reste aliphatique ramifié, saturé ou non saturé, à 3 à 10 atomes de carbone, un reste aliphatique, à chaîne droite ou ramifiée ayant 2 à 10 atomes de carbone, substitué par halogène, alcoxy, ou alkylmercapto, un reste aliphatique à 4 à 10 atomes de carbone substitué par cycloalkyloxy, un reste phényle, éventuellement substitué par halogène, alkyle inférieur, alcoxy inférieur, ou un reste benzyle éventuellement substitué par halogène, allyle, méthallyle, crotyle, 2-éthyl-hexen-2-yle-1, hexen-5-yle-1, 2-méthyl-buten-2-yle-1, 2-méthyl-buten-1-yle-3, butin-1-yle-3, butin-2-yle-1, buten-1-yle-3, propargyle, 2-méthyl-buten-1-yle-4, 2-méthyl-buten-2-yle-4, 3-méthyl-buten-1-yle-3, 1-cyclohexyléthyle,
$R^2$ représente hydrogène, un reste aliphatique à chaîne droite ayant 1 à 10 atomes de carbone, un reste cycloaliphatique à 3 à 7 atomes de carbone, un reste aliphatique ramifié à 3 à 10 atomes de carbone ou un reste alkyle à 2 à 10 atomes de carbone, substitué par halogène ou alcoxy,
X représente oxygène, soufre, un groupe sulfinyle ou sulfonyle et le reste Hal représente un atome d'halogène.

2. Herbicide contenant un composé selon la revendication 1.

3. Herbicide contenant un véhicule solide ou liquide et un composé selon la revendication 1.

4. Procédé de préparation d'un herbicide caractérisé par le fait qu'on mélange un véhicule solide ou liquide avec un composé selon la revendication 1.

5. Procédé pour lutter contre la croissance des plantes indésirables caractérisé par le fait qu'on traite les plantes ou le sol avec un composé selon la revendication 1.

6. Procédé de préparation d'un composé selon la revendication 1, caractérisé par le fait qu'on fait réagir, éventuellement en présence d'un solvant ou diluant et éventuellement en présence d'un accélérateur de réaction, un composé de formule

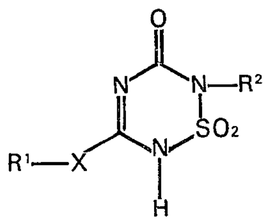

dans laquelle $R^1$, $R^2$ et X ont les significations sus-indiquées, ou ses sels alcalins ou alcalino-terreux, avec un halogénure d'acide phosphorique, acide phosphoreux, acide carbonique, acide oxalique ou acide sulfureux.

**Revendications** pour l'Etat contractant AT

1. Herbicide contenant un composé 6H-1,2,4,6- thiatriazin-1,1-dioxyde de formule

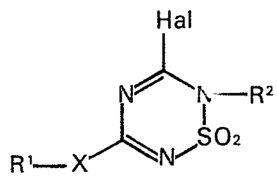

dans laquelle
$R^1$ représente un reste aliphatique saturé, à chaîne droite, ayant 1 à 10 atomes de carbone, un reste cycloaliphatique à 3 à 7 atomes de carbone, un reste aliphatique ramifié, saturé ou non saturé, à 3 à 10 atomes de carbone, un reste aliphatique, à chaîne droite ou ramifiée ayant 2 à 10 atomes de carbone, substitué par halogène, alcoxy, ou alkylmercapto, un reste aliphatique à 4 à 10 atomes de carbone substitué par cycloalkyloxy, un reste phényle, éventuellement substitué par halogène, alkyle inférieur, alcoxy inférieur, ou un reste benzyle éventuellement substitué par halogène, allyle, méthallyle, crotyle, 2-éthyl-hexen-2-yle-1, hexen-5-yle-1, 2-méthyl-buten-2-yle-1, 2-méthyl- buten-1-yle-3, butin-1-yle-3, butin-2-yle-1, buten-1-yle-3, propargyle, 2-méthyl-buten-1-yle-4, 2-méthyl-buten-2-yle-4, 3-méthyl-buten-1-yle-3, 1-cyclohexyléthyle,
$R^2$ représente hydrogène, un reste aliphatique à chaîne droite ayant 1 à 10 atomes de carbone, un reste cycloaliphatique à 3 à 7 atomes de carbone, un reste aliphatique ramifié à 3 à 10 atomes de carbone ou un reste alkyle à 2 à 10 atomes de carbone, substitué par halogène ou alcoxy,
X représente oxygène, soufre, un groupe sulfinyle ou sulfonyle et le reste Hal représente un atome d'halogène.

2. Herbicide contenant un véhicule solide ou liquide et un composé selon la revendication 1.

3. Procédé de préparation d'un herbicide caractérisé par le fait qu'on mélange un véhicule solide ou liquide avec un composé selon la revendication 1.

4. Procédé pour lutter contre la croissance des plantes indésirables caractérisé par le fait qu'on traite les plantes ou le sol avec un composé selon la revendication 1.

5. Procédé de préparation d'un composé selon la revendication 1, caractérisé par le fait qu'on fait réagir, éventuellement en présence d'un solvant ou diluant et éventuellement en présence d'un accélérateur de réaction, un composé de formule

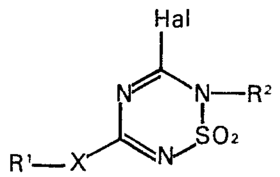

dans laquelle R[1], R[2] et X ont les significations sus-indiquées, ou ses sels alcalins ou alcalino-terreux, avec un halogène d'acide phosphorique, acide phosphoreux, acide carbonique, acide oxalique ou acide sulfureux.